# EUROPÄISCHE PATENTSCHRIFT

(11) **EP 3 981 461 B1**
(45) Veröffentlichungstag und Bekanntmachung des Hinweises auf die Patenterteilung: **19.08.2026**
(21) Anmeldenummer: 20200293.7
(22) Anmeldetag: 06.10.2020
(51) Int. Cl.: A61M 25/02, A61B 1/00, A61B 1/005, A61B 90/57, A61B 90/50

(54) **INSTRUMENT MIT HALTERUNG**
INSTRUMENT WITH HOLDER
INSTRUMENT POURVU DE SUPPORT

(43) Veröffentlichungstag der Anmeldung: 13.04.2022
(73) Patentinhaber: Erbe Elektromedizin GmbH, 72072 Tübingen (DE)
(72) Erfinder: WALZ, Martin, 72076 Tübingen (DE); BEUTLER, Fabian, 72654 Neckartenzlingen (DE); STANEKER, Peter, 72829 Engstingen (DE)
(74) Vertreter: Rüger Abel IP Legal Solutions GmbH

(56) Entgegenhaltungen:
- EP-A1- 0 888 793
- WO-A1-2010/068739
- US-A- 5 364 355
- US-A1- 2003 072 552
- US-A1- 2014 259 544

## Beschreibung

Die Erfindung betrifft ein Instrument zur Behandlung von menschlichen oder tierischen Patienten, insbesondere ein Instrument zur endoskopischen Behandlung.

Prinzipiell sind endoskopisch einsetzbare Sonden zur chirurgischen Behandlung eines Patienten aus dem Stand der Technik bekannt. Beispielsweise offenbart die WO 03/00150 A1 eine Sonde zur Argon-Plasma-Koagulation. Das Instrument weist einen langen Instrumentenkörper mit wenigstens einem flexiblen Abschnitt auf. Der flexible Abschnitt ist zum Einführen in einen Arbeitskanal eines Endoskops geeignet.

Beim Einsatz dieses Instruments an einem Patienten kann es vorkommen, dass das Instrument aus dem Arbeitskanal des Endoskops herausgezogen und temporär beispielsweise in einer entsprechenden sterilen Schale abgelegt werden muss. Dies kann erforderlich werden, um beispielsweise zeitweilig ein anderes Instrument in den Arbeitskanal des Endoskops einzuführen. Bei der temporären Ablage des Instruments in einer sterilen Schale oder an einem anderen geeigneten Ort, darf das Instrument nicht unkontrolliert mit anderen Gegenständen, beispielsweise nicht sterilen Gegenständen oder Flächen in Berührung kommen. Dies ist aufgrund der Länge des Instruments, die zum Beispiel zwei Meter übersteigen kann, nicht ohne weiteres von vornherein sicherzustellen. Beispielsweise kann es zweckmäßig sein, das Instrument in mehreren Windungen zusammenzulegen. Aufgrund der ihm typischerweise eigenen Federelastizität hat es jedoch die Neigung, zurück in eine gestreckte Form zu springen. Aus der US 5 372 592 A sowie aus der WO 2010/068739 A1 ist es dazu bekannt, das zusammengelegte Instrument mit einer Klemme zu fixieren. Gemäß US 2012/0220897 A1 kann die Klemme nach Art einer Schraubenfeder ausgebildet sein, die an einem Ende eine sich spiralförmig erweiterte Windung aufweist. Allerdings ist die Handhabung mühsam und stellt ein Hygienerisiko dar. Die US 5 364 355 schlägt vor, eine mit einer Schraubennut versehene Hülse längsbeweglich und drehbar auf einem proximalen Abschnitt eines flexiblen Instruments anzuordnen. Die EP 0 888 793 A1 schlägt vor, an dem proximalen Ende eines Instruments eine Klemme mit einem beweglichen Bügel anzuordnen, der zwischen einer offenen Position und einer geschlossenen Position hin und her schwenkbar ist. Dagegen nutzen die US 2014/0259544 A1**,** die US 2008/0264993 A1 und die WO 2010/068739 A1 feste, unbeweglich am Instrument gehaltene Klemmen zur Festlegung von Windungen eines zusammengelegten Instruments. Zur Aufnahme eines Lichtleiters schlägt die US 2003/0072552 A1 einen Halter vor, der ein mit zwei Befestigungslöchern versehenes Ende und einen sich daran anschließenden Abschnitt mit einem zu einer Spirale gebogenen Blechstreifen aufweist. In diesen kann der Lichtleiter eingelegt werden.

Es ist Aufgabe der Erfindung, ein verbessertes Konzept zur Handhabung des Instruments insbesondere bei Nichtbenutzung desselben anzugeben.

Diese Aufgabe wird mit dem Instrument nach Anspruch 2 mit einer Halteeinrichtung nach Anspruch 1 gelöst.

Das erfindungsgemäße Instrument ist insbesondere zum endoskopischen Einsatz vorgesehen. Es handelt sich bei dem Instrument beispielsweise um eine Sonde, die in einen Arbeitskanal eines Endoskops eingeführt und mit dem abwinkelbaren Ende des Endoskops in verschiedene Richtungen bewegt werden kann. Dazu weist das Instrument einen Instrumentenkörper mit wenigstens einem flexiblen Abschnitt auf. Der flexible Abschnitt kann zum Beispiel über 1 m lang sein und in wenigstens einer vorzugsweise mehreren Windungen aufgewickelt werden. Zu dem Instrument gehört weiter eine an dem Instrumentenkörper angeordnete Halteeinrichtung, die die Windung(en) aufnehmen und fixieren kann. Der Instrumentenkörper kann durch einen ein- oder mehrlumigen Schlauch mit zylindrischer Außenkontur, d.h. kreisförmigem Querschnitt gebildet sein. Der Instrumentenkörper kann auch einen nichtkreisförmigen, z.B. ovalen Querschnitt aufweisen oder aus zwei oder mehreren zueinander parallel angeordneten Schläuchen bestehen.

Durch die Anordnung der Halteeinrichtung an dem Instrumentenkörper kann die Halteeinrichtung mit einer Hand, nämlich derjenigen Hand, bedient werden, die den Instrumentenkörper hält. Durch die anspruchsgemäße Ausbildung der Halteeinrichtung baut diese axial kurz und ist dadurch leicht zu bedienen. Mit der anderen Hand kann die Bedienperson den Instrumentenkörper in einer oder mehreren Windungen zusammenlegen und mit einer seiner Hände die Halteeinrichtung betätigen, um die Windungen aufzunehmen und zu fixieren. Mit diesem Konzept wird eine einfache Handhabung erreicht und es werden Gefahren für die Sterilität des Instruments beseitigt. Die an dem Instrumentenkörper angeordnete Halteeinrichtung ist so steril wie der Instrumentenkörper selbst. Nachdem die Halteeinrichtung untrennbar mit dem Instrumentenkörper verbunden ist, kann sie nicht abseits von demselben abgelegt werden. Kontaminationsgefahren werden minimiert.

Mit der Erfindung wird das temporäre Ablegen des Instruments beim Instrumentenwechsel erleichtert. Das Instrument kann schnell und auf einfache Weise platzsparend zusammengelegt und fixiert werden. Instrumente mit Längen von über 2 m können auf diese Weise sicher gehandhabt werden, ohne dass die zusammengelegten Windungen aufspringen und beispielsweise zu Boden fallen.

Der Instrumentenkörper kann aus einem Zuleitungsabschnitt und einem Sondenabschnitt bestehen. Der Sondenabschnitt und der Zuleitungsabschnitt können unterschiedliche Durchmesser aufweisen, aus unterschiedlichen Materialien bestehen und/oder unterschiedliche Flexibilitäten haben. Der Instrumentenkörper kann jedoch auch durchgehend durch einen Schlauch gleichmäßiger Dicke und/oder aus ein und demselben Material gebildet sein. Auch in diesem Fall kann jedoch derjenige Teil des Instrumentenkörpers, der in ein Endoskop einzusetzen ist, als Sondenabschnitt angesehen werden. Derjenige Teil des Instrumentenkörpers, der bei einer Behandlung außerhalb des Endoskops verbleibt, kann als Zuleitungsabschnitt angesehen werden. Die Halteeinrichtung ist vorzugsweise auf dem Zuleitungsabschnitt angeordnet. Sie kann dort axial unverschiebbar, d.h. gegen axiale Verschiebung gesichert gehalten sein. Alternativ kann die Halteeinrichtung auf dem Sondenkörper auch axial begrenzt verschiebbar angeordnet sein. Z.B. kann der axiale Bewegungsspielraum auf beiden Seiten durch Stop-Elemente begrenzt sein. Ein Stop-Element kann an einer Seite durch den Handgriff des Instruments gebildet sein. Außerdem kann ein Stop-Element an einer Seite oder an beiden Seiten durch ein Gummielement, z.B. einen O-Ring gebildet sein.

Das Instrument kann zum Beispiel eine Argon-Plasma-Sonde sein, die einen sich vom proximalen Ende zum distalen Ende erstreckenden mit Gas beaufschlagbaren Kanal und eine darin angeordnete Elektrode aufweist. Das Instrument kann jedoch auch auf ein anderweitiges Instrument beispielsweise ein Instrument zur Kryobehandlung, ein faseroptisches Instrument zur Laserbehandlung oder dergleichen sein. Allen solchen Instrumenten ist jedoch gemeinsam, dass sie einen schlanken, langen Instrumentenkörper aufweisen, der wenigstens abschnittsweise flexibel ist, so dass er zu einer oder mehreren Windungen zusammengelegt werden kann.

Vorzugsweise weist die Halteeinrichtung einen bezüglich des Instrumentenkörpers drehbar gelagerten Greifer auf. Der Greifer ist vorzugsweise ein einstückiges steifes Kunststoffteil ohne bewegliche Abschnitte. Die Drehachse des Greifers ist dabei vorzugsweise mit der Längsrichtung des Instrumentenkörpers identisch oder parallel zu dieser. Dies ermöglicht eine besonders leichte ergonomische Bedienung durch den Benutzer. Dieser kann nämlich mit den Fingern der Hand, in der er den Instrumentenkörper hält, zugleich den starren Greifer der Halteeinrichtung drehen. Der Greifer kann dadurch eine oder mehrere Windungen des zusammengelegten und womöglich mit der anderen Hand des Bedieners gehaltenen Instrumentenkörpers aufnehmen und fixieren.

Dazu ist es besonders vorteilhaft, wenn der Greifer einen um den Instrumentenkörper, insbesondere einen um die Längsachse desselben gekrümmten Aufnahmeraum für den Sondenabschnitt aufweist. Der Greifer ist somit mit Blickrichtung parallel zur Längsrichtung des Sondenkörpers hakenförmig ausgebildet. Der Aufnahmeraum ist dabei entlang eines bogenförmigen, um die Drehachse des Greifers gekrümmten Wegs vorzugsweise länger als der drittel Umfang des Greifers, besonders bevorzugt länger als der halbe Umfang des Greifers. Dadurch können sicher mehrere Windungen des Instrumentenkörpers erfasst und gehalten werden.

An der Halteeinrichtung können ein oder mehrere Strukturen zur Aufnahme eines Abschnitts des Instrumentenkörpers, insbesondere seines distalen Endabschnitts vorgesehen sein. Diese Strukturen können dazu dienen, das distale Ende des Instrumentenkörpers festzulegen, so dass dieses bei dem kompakt zusammengelegten Instrumentenkörper bleibt. Dabei gelingt es insbesondere, das distale Ende des Instrumentenkörpers vor Verschmutzung und damit den Patienten davor zu schützen, mit Keimen oder Schmutz in Berührung zu kommen.

Strukturen zur wenigstens zeitweiligen Aufnahme und Lagerung des distalen Endes des Instrumentenkörpers können ein oder mehrere Rastausnehmungen sein, die an dem Sockel der Halteeinrichtung oder an dem drehbar gelagerten Greifer angeordnet sind. Solche Rastausnehmungen können C-förmige Ausnehmungen sein, in die der Sondenschlauch einrastbar ist. Die Ausbildung solcher Rastausnehmungen in dem Greifer hat den besonderen Vorteil, dass mit dem Einrasten des distalen Endes oder eines sonstigen Abschnitts des Instrumentenkörpers in die Rastausnehmung auch die weitere Drehung des Greifers blockiert ist und Windungen des Instruments in dem Greifer gesichert sind. Das in der Rastausnehmung gehaltenen Ende des Instruments versperrt den Windungen den Weg aus dem Greifer heraus.

Weitere Einzelheiten vorteilhafter Ausführungsformen und Varianten der Erfindung sind Gegenstand von Unteransprüchen sowie der Figuren, der Zeichnung und der zugehörigen Beschreibung. Es zeigen:
Figur 1 ein Endoskop mit einem darin eingesetzten Instrument, in schematischer Gesamtdarstellung,
Figur 2 das aus dem Endoskop entnommene zusammengelegte Instrument, in Gesamtdarstellung,
Figur 3 das an seiner Halteeinrichtung geschnittene Instrument, in Querschnittsansicht,
Figur 4 die Halteeinrichtung, in perspektivischer Explosionsdarstellung,
Figur 5 das Instrument und die Halteeinrichtung, in ausschnittsweiser Längsschnittdarstellung,
Figur 6 einen zu der Halteeinrichtung gehörenden Sockel, in Stirnansicht,

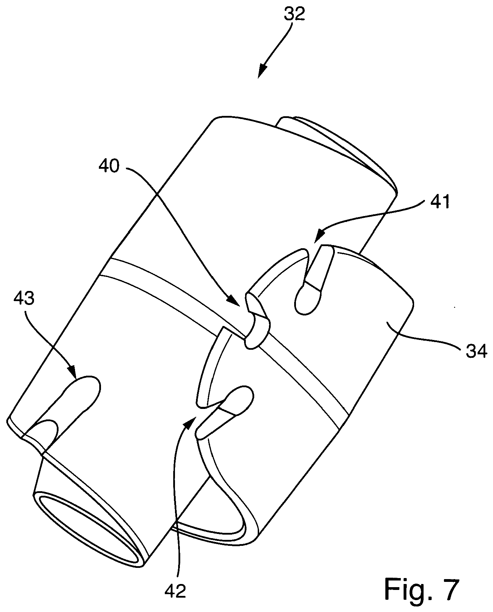

Figur 7 einen zu der Halteeinrichtung gehörigen Greifer, in Perspektivdarstellung,
Figur 8 den Greifer nach Figur 7, in einer abgewandelten Perspektivdarstellung,
Figur 9 eine abgewandelte Ausführungsform des der Halteeinrichtung für ein Instrument mit einer Doppelschlauchanordnung im Zuleitungsbereich,
Figur 10 ein Instrument mit zwei Schläuchen im Zuleitungsbereich und Halteeinrichtung mit Sockel
Figur 11 ein Instrument mit einer sockellosen Halteeinrichtung in Querschnittsdarstellung, geschnitten in Nachbarschaft seiner Halteeinrichtung,

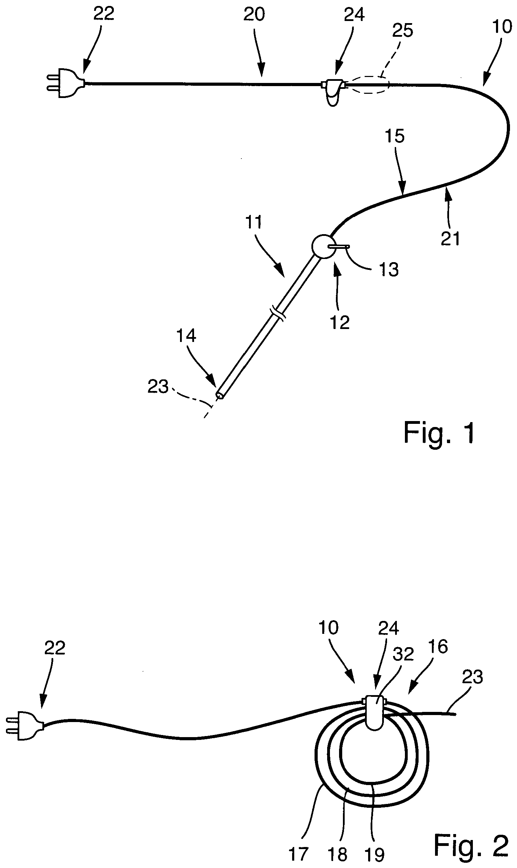

In Figur 1 ist ein Instrument 10 veranschaulicht, das als flexible Sonde ausgebildet und zum Einsatz in einem Endoskop 11 vorgesehen ist. Das Endoskop 11 weist einen Arbeitskanal auf, durch den sich das Instrument 10 erstreckt. Außerdem weist das Endoskop 11 an seinem proximalen Ende 12 wenigstens ein Bedienorgan 13 auf, das dazu dient, das distale Ende 14 des Endoskops zu steuern, beispielsweise seitlich abzuwinkeln. Das flexible Instrument 10 folgt dieser Bewegung.

Bei dem Instrument 10 kann es sich um jedes zur Behandlung eines menschlichen oder tierischen Patienten geeignete Instrument handeln. Es kann sich insbesondere um ein kryochirurgisches Instrument, ein elektrochirurgisches Instrument, ein plasmachirurgisches Instrument oder auch ein Instrument zur Behandlung mit Radiowellen oder mit Licht, beispielsweise ein faseroptisches Instrument zur Laserbehandlung von biologischem Gewebe handeln. Wesentlich ist im Zusammenhang mit der vorliegenden Erfindung, dass das Instrument 10 einen Instrumentenkörper 15 mit einem flexiblen Abschnitt 16 aufweist, der sich, wie Figur 2 veranschaulicht, zu ein oder mehreren Windungen 17, 18, 19 zusammenlegen lässt.

Der Instrumentenkörper 15 kann beispielsweise durch einen Zuleitungsabschnitt 20 und einen Sondenabschnitt 21 gebildet sein. Beide Abschnitte 20, 21 können beispielsweise aus einem ein- oder mehrlumigen Schlauch bestehen. Das Material des Zuleitungsabschnitts 20 kann das gleiche Material wie das Material des Sondenabschnitts 21 sein. Es können jedoch auch unterschiedliche Materialien Anwendung finden. Jedoch bestehen sowohl der Zuleitungsabschnitt 20 als auch der Sondenabschnitt 21 vorzugsweise aus Kunststoff. Der Zuleitungsabschnitt 20 und der Sondenabschnitt 21 können einen runden Querschnitt und gleiche oder unterschiedliche Durchmesser aufweisen. Ebenso können der Zuleitungsabschnitt 20 und der Sondenabschnitt 21 gleiche oder unterschiedliche Flexibilitäten aufweisen. Es ist auch möglich, den Zuleitungsabschnitt 20 durch einen einzigen durchgehend ausgebildeten Körper zu bilden.

An dem proximalen Ende des Instruments 10 können Anschlussmittel, wie beispielsweise ein Stecker 22, vorgesehen sein, über die das Instrument 10 mit Strom und/oder Betriebsmedien, Licht oder anderen Energieformen versorgbar ist. Das distale Ende 23 dient hingegen der Einwirkung auf biologisches Gewebe am oder im Patienten.

Erfindungsgemäß weist das Instrument 10 eine Halteeinrichtung 24 auf, die dazu dient, Windungen 17, 18, 19 des Instruments 10 wie in Figur 2 veranschaulicht zusammenzuhalten. Die Halteeinrichtung 24 kann, wie Figur 1 veranschaulicht, zum Beispiel in der Nähe eines optionalen und deswegen in Figur 1 gestrichelt dargestellten Handgriffs 25 angeordnet sein. Dieser dient der Handhabung des Instruments 10 insbesondere beim Einführen in das Endoskop 11 und/oder während der Behandlung am Patienten. Der Handgriff 25 kann beispielsweise an einer Stelle des Instruments 10 angeordnet sein, an dem sich der Zuleitungsabschnitt 20 und der Sondenabschnitt 21 treffen.

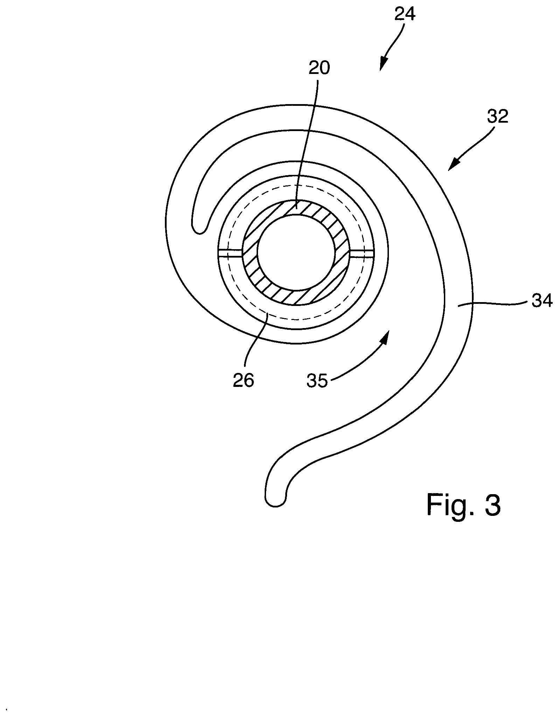

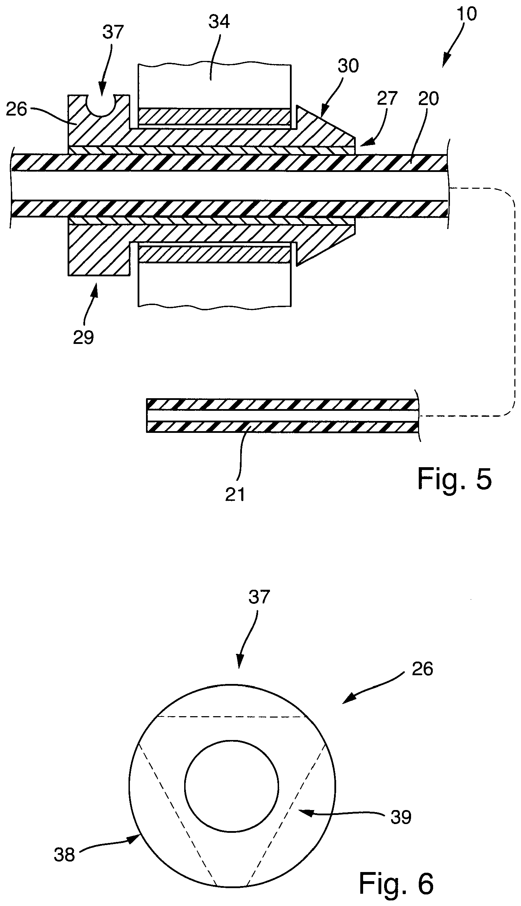

Die vorzugsweise auf dem Zuleitungsabschnitt 20 angeordnete Halteeinrichtung 24 ist in den Figuren 3, 4 und 5 veranschaulicht. Die Halteeinrichtung 24 kann einen z.B. aus Kunststoff ausgebildeten Sockel 26 aufweisen, der auf dem Instrument 10, beispielsweise auf dem Zuleitungsabschnitt 20, angeordnet ist. Der Sockel 26 kann, wie Figur 5 andeutet, auf dem Instrument 10, zum Beispiel auf seinem Zuleitungsabschnitt 20, angeordnet sein. Der Sockel 26 kann dabei auf dem Zuleitungsabschnitt 20 festgeklemmt oder festgeklebt oder auf anderer Weise gegen axiales Verschieben gesichert angeordnet sein. Rein beispielhaft ist dazu in Figur 5 eine Klebstoffschicht 27 dargestellt. Alternativ kann der Sockel 26 beispielsweise durch Gummielemente, zum Beispiel Gummiringe, reibschlüssig auf der Außenfläche des Instruments 10 gehalten sein. Es ist auch möglich, den Sockel 26 axial verschiebbar mit Spiel auf dem Instrument 10, insbesondere dem Zuleitungsabschnitt 20, anzuordnen. Der Sockel 26 kann allerdings auch weggelassen werden.

Der Sockel 26 weist vorzugsweise einen außen zylindrischen Lagerabschnitt 28 auf, an dem sich ein radial nach außen überstehender Kopf 29 anschließt. An dem anderen Ende des Lagerabschnitts 28 ist ein Rastbund 30 vorgesehen, der mit einer Ringfläche den Lagerabschnitt 28 axial begrenzt. Längsschlitze 31, die sich bis in den Lagerabschnitt 30 erstrecken können, teilen den Rastbund 30 dessen Hälften so radial einwärts federn können.

Unabhängig davon, ob die Halteeinrichtung 24 einen Sockel 26 aufweist oder nicht, gehört ein Greifer 32 zu der Halteeinrichtung 24, der drehbar an dem Instrument 10 gehalten ist. Z.B. kann der Greifer auf dem Lagerabschnitt 28 gehalten sein. Alternativ kann der Greifer 32 auch direkt auf dem Instrument 10 drehbar gehalten sein. Der Greifer 32 ist vorzugsweise ein Kunststoffteil. Es weist beispielsweise ein rohrförmiges Mittelstück 33 auf, von dem sich eine haken- oder spiralförmig geformte Zunge 34 weg erstreckt. Das Mittelstück 33 kann wie eine Buchse mit geschlossener Wandung ausgebildet sein. Alternativ kann das Mittelstück 33 einen Längsschlitz aufweisen, um in einer Rastbewegung an dem Instrument 10 oder dem Sockel 26 angebracht zu werden.

Die Zunge 34 windet sich dabei in einem Abstand zu dem Mittelstück 33 um diese herum. So begrenzt die Zunge 34 zusammen mit dem rohrförmigen Mittelstück 33 einen gebogenen Aufnahmeraum 35, dessen Weite (d.h. der Abstand zu dem Mittelstück 33) und Tiefe ausreicht, um die Windungen 17, 18, 19 zum Beispiel des Sondenabschnitts 21 aufzunehmen. Der Aufnahmeraum 35 erstreckt sich bogenförmig um das rohrförmige Mittelstück 33 herum und führt dabei vorzugsweise um mehr als eine halbe Umdrehung, d.h., mehr als 180° um die Mittelachse 36.

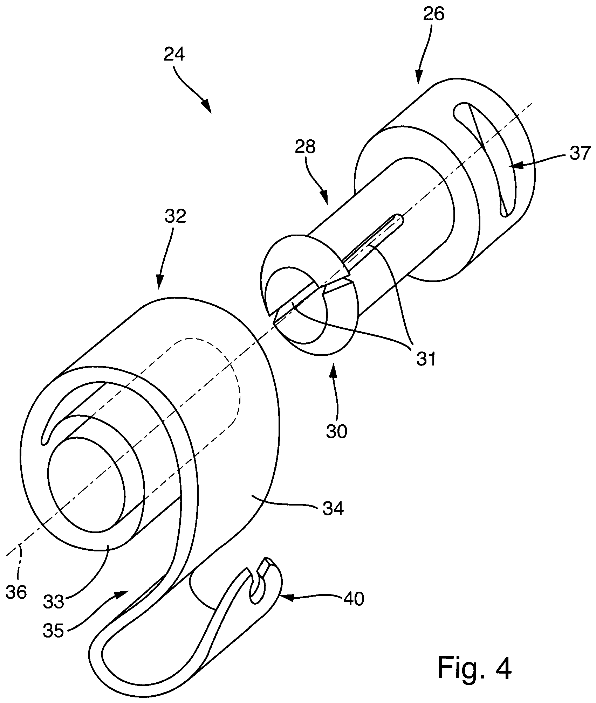

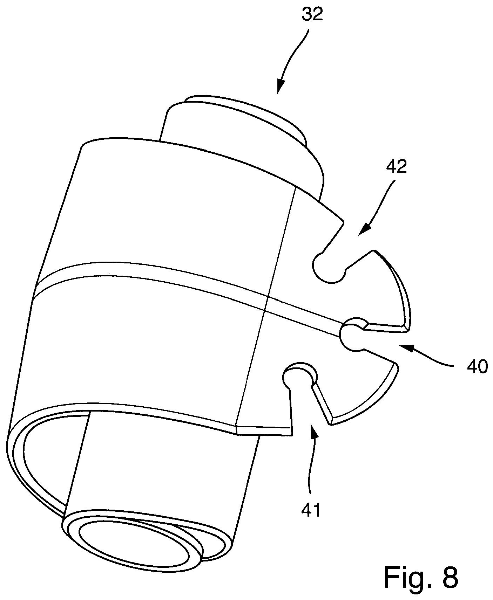

An dem Sockel 26 können ein oder mehrere Rastausnehmungen 37, 38, 39 ausgebildet sein, wie insbesondere aus den Figuren 4, 5 und 6 hervorgeht. Vorzugsweise sind diese Rastausnehmungen 37 bis 39 in dem Kopf 29 des Sockels 26, beispielsweise in Gestalt gerader Nuten ausgebildet, die in ihrem Mittelbereich etwas enger ist als der Durchmesser des Sondenabschnitts 20. Das distale Ende 23 oder der sich daran in proximaler Richtung anschließende Teil des Sondenschlauchs 21 kann somit in diese Rastausnehmungen 37 bis 39 eingerastet werden und ist dadurch temporär festgehalten. Es ist aber auch möglich, eine Rastausnehmung 40 an dem freien Ende der Zunge 34 auszubilden, wie aus Figur 4 hervorgeht. Das distale Ende 23 des Sondenabschnitts 21 oder ein sich daran anschließender Teil desselben kann in diese Rastausnehmung 40 eingesetzt werden. Es ist auch möglich, an dem freien Ende der Zunge 34 des Greifers 32 mehrere solcher Rastausnehmungen 40, 41, 42 auszubilden. Ebenso kann in der Flanke der Zunge 34 eine weitere Rastausnehmung 43 angeordnet sein. Solche Rastausnehmungen 40 bis 42 gehen außerdem aus Figur 8 hervor.

Das insoweit beschriebene Instrument 10 kann beispielsweise in der in Figur 2 dargestellten zusammengelegten Form steril verpackt von einem Instrumentenhersteller bereitgestellt und geliefert werden, die Windungen 17, 18, 19 können durch Drehung des Greifers 32 befreit werden, sodass das Instrument 10 einerseits mit einem speisenden Gerät verbunden und andererseits in den Arbeitskanal eines Endoskops 11 eingeführt werden kann,

Muss das Instrument 10 aus dem Arbeitskanal des Endoskops 11 während der Operation herausgenommen und zwischenabgelegt werden, kann es leicht wieder, wie in Figur 2 veranschaulicht, zusammengelegt werden, indem insbesondere sein Sondenabschnitt 21 in mehreren Windungen 17, 18, 19 zusammengelegt wird. Dies ist mit zwei Händen ohne weiteres möglich, wobei mit einer der Hände der Greifer 32 so gedreht werden kann, dass er die Windungen 17, 18, 19 fasst und sichert. Bedarfsweise kann nun noch das distale Ende 23 in einer der Rastausnehmungen 37 bis 43 gesichert werden. Werden die Rastausnehmungen 40, 41, 42 oder auch die Rastausnehmung 43 genutzt, kann dadurch eine Rückdrehung des Greifers 32 blockiert werden. Dies ist eine zusätzliche Sicherheit gegen das Aufspringen der Windungen 17, 18, 19. Das somit sicher zusammengelegte Instrument 10 kann nun zum Beispiel in einer sterilen Schale zwischenabgelegt werden, ohne dass die Gefahr unkontrollierter Bewegungen und Kontaminationen besteht.

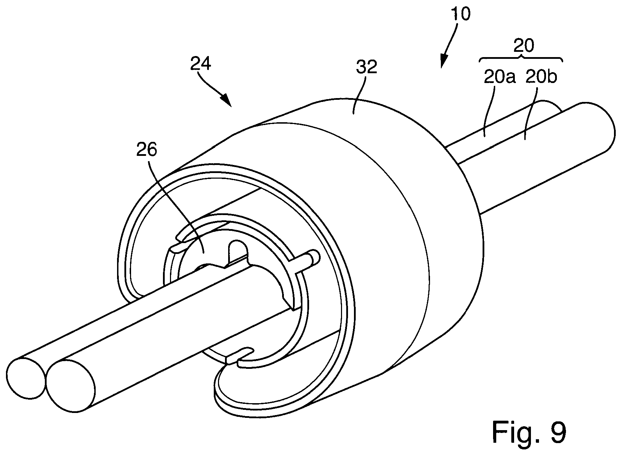

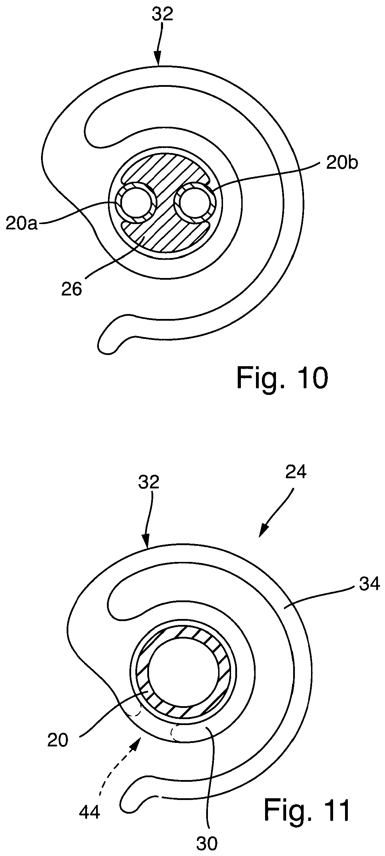

Weiter ist aus Figur 9 ein Instrument 10 ersichtlich, das in seinem Zuleitungsabschnitt 20 zwei Leitungen 20a, 20b aufweist. Der Sockel 26 ist dazu eingerichtet, beide Leitungen 20a, 20b aufzunehmen. Er muss diese jedoch nicht auf ganzem Umfang umschließen, wodurch sich eine schlankere Bauform erreichten lässt. Eine ähnliche Bauform ergibt sich aus Figur 10. Auch dort ist zur Aufnahme beider Leitungen 20a, 20b ein Sockel 26 vorgesehen, der mit den beiden Leitungen 20a, 20b wie bei der Ausführungsform nach Figur 9 verklebt sein kann oder in Rastverbindung mit diesen steht. Hinsichtlich der Ausführung des Greifers 32 gelten die zu allen anderen Ausführungsformen gegebenen Beschreibungen entsprechend.

In Figur 11 ist eine Ausführungsform der Halteeinrichtung 24 erkennbar, bei der der Greifer 32 ohne Sockel direkt auf dem Zuleitungsabschnitt 20 oder auch auf einem anderen Abschnitt des Instruments 10 gehalten ist. Er ist dabei um die Längsachse des Zuleitungsabschnitts 20 drehbar, d.h., er ist mit etwas Radialspiel auf dem Zuleitungsabschnitt 20 gehalten. Außerdem ist der Greifer 32 axial auf dem Zuleitungsabschnitt 20 verschiebbar. Zur Begrenzung des Verschiebewegs können entsprechende nicht weiter dargestellte Stopp-Elemente vorgesehen sein. Stopp-Elemente können zum Beispiel auf dem Zuleitungsabschnitt 20 angebrachte O-Ringe, durch Clip-Element oder auch durch an dem Instrument 10 natürlicherweise vorhandene Strukturen, wie zum Beispiel der Handgriff gebildet sein. Der Rohrabschnitt 30 kann, wie Figur 11 veranschaulicht, als geschlossener Hohlzylinder wie eine Buchse ausgebildet sein. Alternativ ist es aber auch möglich, in dem Mittelabschnitt 30 eine gegebenenfalls mit gerundeten Flanken versehenen Schlitz 44 auszubilden, wie in Figur 11 gestrichelt dargestellt ist. Dies hat den Vorteil, dass der Greifer 32 dann auf die entsprechende Lagerstruktur, d.h. einen Sockel oder auch den Zuleitungsabschnitt 20 oder einen anderen Teil des Instruments 10 aufgerastet werden kann. Damit eignet sich der Greifer 32 auch zur nachträglichen Befestigung auf einem Instrument 10, das zuvor nicht mit einer Halteeinrichtung 24 ausgerüstet war. Der Schlitz 44 kann bei allen vorbeschriebenen Ausführungsformen Anwendung finden.

Jede der vorbeschriebenen oder im Zusammenhang mit dem Instrument 10 nachstehend beanspruchten Halteeinrichtungen 24 kann auch unabhängig von dem Instrument 10 bereitgestellt und in Verkehr gebracht werden. In solchen Fällen ist die Halteeinrichtung 24 nachträglich an dem Instrument 10 anzubringen.

Das erfindungsgemäße Instrument 10 weist einen Instrumentenkörper 15 auf, der insbesondere lang, schlank und flexibel ausgebildet ist. Er kann ganz oder teilweise in wenigstens einer oder mehreren Windungen 17, 18, 19 aufgewickelt werden und auf diese Weise platzsparend und gut handhabbar zusammengelegt werden. Um den Instrumentenkörper 15 in dieser Position zu halten, ist eine Halteeinrichtung 24 vorgesehen, die auf dem Instrumentenkörper 15 angeordnet und dort vorzugsweise unverlierbar gehalten ist. Die Halteeinrichtung weist einen um den Instrumentenkörper herum drehbeweglich angeordneten Greifer 32 auf, mit einer Zunge 34, die die Windungen 17, 18, 19 aufnehmen und fixieren kann. Der Greifer 32 kann mit den Fingern derjenigen Hand betätigt werden, mit der der Bediener das Instrument 10 hält.

### Bezugszeichen:

- 10: Instrument
- 11: Endoskop
- 12: proximales Ende des Endoskops
- 13: Bedienorgan
- 14: distales Ende des Endoskops
- 15: Instrumentenkörper des Instruments 10
- 16: flexibler Abschnitt des Instruments 10
- 17 - 19: Windungen
- 20: Zuleitungsabschnitt
- 20a, 20b: Leitungen
- 21: Sondenabschnitt
- 22: Anschlussmittel des Instruments 10
- 23: distales Ende des Instruments 10
- 24: Halteeinrichtung
- 25: Handgriff
- 26: Sockel
- 27: Klebstoff
- 28: Lagerabschnitt
- 29: Kopf
- 30: Rastbund
- 31: Längsschlitze
- 32: Greifer
- 33: Mittelstück
- 34: Zunge
- 35: Aufnahmeraum
- 36: Mittelachse
- 37 - 43: Rastausnehmungen
- 44: Schlitz

## Patentansprüche

1. Halteeinrichtung (24) für ein Instrument (10) zur medizinischen Behandlung von menschlichen oder tierischen Patienten, insbesondere zur endoskopischen Behandlung,
wobei die Halteeinrichtung (24) einen an einem Instrumentenkörper (15) drehbeweglich anordenbaren Greifer (32) aufweist, um eine Windung (17) des Instruments (10) aufzunehmen,
wobei dass der Greifer (32) ein rohrförmiges Mittelstück (33) aufweist, von dem sich eine Zunge (34) in radialem Abstand zu dem Mittelstück (33) um wenigstens einen Teil des Umfangs des Mittelstücks (33) weg erstreckt,
**dadurch gekennzeichnet,**
**dass** der Greifer (32) ein einstückiges starres Kunststoffteil ist, der auf einem Sockel (26) angeordnet und drehbar gelagert ist, wobei der Greifer (32) einen um den Instrumentenkörper (15) gekrümmten Aufnahmeraum (35) für den Sondenabschnitt (21) aufweist, der in Drehrichtung des Greifers (32) länger als eine Drittel-Umdrehung des Greifers (32) ist.

2. Instrument (10) zur medizinischen Behandlung von menschlichen oder tierischen Patienten, insbesondere zur endoskopischen Behandlung,
mit einem Instrumentenkörper (15), der wenigstens einen flexiblen, in wenigstens einer Windung (17) aufwickelbaren Abschnitt (16) aufweist, wobei der Instrumentenkörper (15) aus einem Zuleitungsabschnitt (20) und einem Sondenabschnitt (21) besteht, wobei der Sondenabschnitt (21) flexibel und in wenigstens einer, vorzugsweise mehreren Windungen (17, 18, 19) aufwickelbar ist,
mit einer Halteeinrichtung (24) nach Anspruch 1, die an dem Instrumentenkörper (15) drehbeweglich angeordnet ist.

3. Halteeinrichtung nach Anspruch 1, **dadurch gekennzeichnet, dass** der Greifer (32) wenigstens eine Rastausnehmung (40) zur Aufnahme eines Abschnitts (23) des Instrumentenkörpers (15) aufweist.

4. Halteeinrichtung nach Anspruch 1, **dadurch gekennzeichnet, dass** der Sockel (26) wenigstens eine Rastausnehmung (37) zur Aufnahme eines Abschnitts (23) des Instrumentenkörpers (15) aufweist.

5. Halteeinrichtung nach einem der vorstehenden Ansprüche, **dadurch gekennzeichnet, dass** die Halteeinrichtung (24) auf dem Instrumentenkörper (15) unverschiebbar gesichert ist.

6. Halteeinrichtung nach einem der vorstehenden Ansprüche, **dadurch gekennzeichnet, dass** die Halteeinrichtung (24) an dem Instrumentenkörper (15) unverlierbar gehalten ist.

7. Halteeinrichtung nach einem der vorstehenden Ansprüche, **dadurch gekennzeichnet, dass** die Halteeinrichtung (24) einen Sockel (26) umfasst, der zur Befestigung an einem Instrument mit nichtrundem Querschnitt eingerichtet ist.

8. Instrument nach Anspruch 2 und Halteeinrichtung nach Anspruch 6, **dadurch gekennzeichnet, dass** der Sockel (26) den Zuleitungsabschnitt (20) oder den Sondenabschnitt (21) auf ganzem Umfang umgreift oder, alternativ, lediglich auf einem Teil seines Umfangs umgreift.

9. Instrument nach Anspruch 2 und Halteeinrichtung nach einem der vorstehenden Ansprüche, **dadurch gekennzeichnet, dass** die Halteeinrichtung (24) axial beweglich auf dem Zuleitungsabschnitt (20) oder dem Sondenabschnitt (21) angeordnet ist.

## Claims

1. Holding device (24) for an instrument (10) for medical treatment of human or animal patients, particularly for endoscopic treatment,
wherein the holding device (24) comprises a gripper (32) that can be rotatably arranged on an instrument body (15), for receiving a winding (17) of the instrument (10),
wherein the gripper (32) has a tube-shaped center part (33) from which a latch (34) extends away with radial distance to the center part (33) around at least a part of the circumference of the center part (33),
**characterized in that**
the gripper (32) is a one-piece rigid plastic part that is arranged on a base (26) and rotatably supported, wherein the gripper (32) comprises a holding space (35), curved around the instrument body (15), for the probe section (21), the holding space (35) being longer in the rotation direction of the gripper (32) than one third of a rotation of the gripper (32).

2. Instrument (10) for medical treatment of human or animal patients, particularly for endoscopic treatment,
having an instrument body (15) that comprises at least one flexible section (16) that can be wound in at least one winding (17), wherein the instrument body (15) consists of a supply section (20) and a probe section (21),
wherein the probe section (21) is flexible and can be wound in at least one, preferably multiple, windings (17, 18, 19),
having a holding device (24) according to claim 1, which is arranged in a rotatable manner on the instrument body (15).

3. Holding device according to claim 1, **characterized in that** the gripper (32) comprises at least one latch recess (40) for receiving a section (23) of the instrument body (15).

4. Holding device according to claim 1, **characterized in that** the base (26) comprises at least one latch recess (37) for receiving a section (23) of the instrument body (15).

5. Holding device according to any of the preceding claims, **characterized in that** the holding device (24) is secured on the instrument body (15) in a non-displaceable manner.

6. Holding device according to any of the preceding claims, **characterized in that** the holding device (24) is undetachably held on the instrument body (15).

7. Holding device according to any of the preceding claims, **characterized in that** the holding device (24) comprises a base (26) that is configured for attachment to an instrument having a non-round cross-section.

8. Instrument according to claim 2 and holding device according to claim 6, **characterized in that** the base (26) surrounds the supply section (20) or the probe section (21) around the whole circumference or, alternatively, surrounds it only around part of its circumference.

9. Instrument according to claim 2 and holding device according to any of the preceding claims, **characterized in that** the holding device (24) is axially movably arranged on the supply section (20) or the probe section (21).

## Revendications

1. Dispositif de support (24) pour un instrument (10) destiné au traitement médical de patients humains ou d'animaux, en particulier au traitement endoscopique,
dans lequel le dispositif de support (24) présente une pince (32) pouvant être disposée de façon mobile en rotation sur un corps d'instrument (15) afin de saisir une spire de l'instrument (10),
la pince (32) présentant une partie centrale (33) tubulaire à partir de laquelle s'étend une languette (34) autour d'au moins une partie de la circonférence de la partie centrale (33), à distance radiale de la partie centrale (33),
**caractérisé en ce que**
la pince (32) est un élément en matière plastique rigide, réalisé d'une seule pièce, qui est disposé sur un socle (26) et est monté mobile en rotation, la pince (32) présentant un espace (35) incurvé autour du corps d'instrument (15) et destiné à accueillir la partie de sonde (21), qui présente une longueur, dans le sens de rotation de la pince (32), qui est supérieure à un tiers de tour de la pince (32).

2. Instrument (10) destiné au traitement médical de patients humains ou d'animaux, en particulier au traitement endoscopique,
comprenant un corps d'instrument (15) qui présente au moins une partie (16) flexible pouvant être enroulée en au moins une spire (17), le corps d'instrument (15) étant constitué d'une partie d'alimentation (20) et d'une partie de sonde (21), la partie de sonde (21) étant flexible et pouvant être enroulée en au moins une spire, de préférence en plusieurs spires (17, 18, 19),
comprenant un dispositif de support (24) selon la revendication 1, qui est installé de façon mobile en rotation sur le corps d'instrument (15).

3. Dispositif de support selon la revendication 1, **caractérisé en ce que** la pince (32) présente au moins une encoche d'encliquetage (40) destinée à accueillir une partie (23) du corps d'instrument (15).

4. Dispositif de support selon la revendication 1, **caractérisé en ce que** le socle (26) présente au moins une encoche d'encliquetage (37) destinée à accueillir une partie (23) du corps d'instrument (15).

5. Dispositif de support selon une des revendications précédentes, **caractérisé en ce que** le dispositif de support (24) est bloqué sans possibilité de déplacement sur le corps d'instrument (15).

6. Dispositif de support selon une des revendications précédentes, **caractérisé en ce que** le dispositif de support (24) est tenu de manière imperdable sur le corps d'instrument (15).

7. Dispositif de support selon une des revendications précédentes, **caractérisé en ce que** le dispositif de support (24) comporte un socle (26) qui est agencé en vue de la fixation à un instrument de section non circulaire.

8. Instrument selon la revendication 2 et dispositif de support selon la revendication 6, **caractérisés en ce que** le socle (26) entoure la partie d'alimentation (20) ou la partie de sonde (21), sur la totalité de la circonférence ou, de manière alternative, seulement sur une partie de sa circonférence.

9. Instrument selon la revendication 2 et dispositif de support selon une des revendications précédentes, **caractérisés en ce que** le dispositif de support (24) est installé de manière axialement mobile sur la partie d'alimentation (20) ou la partie de sonde (21).
